# EUROPEAN PATENT SPECIFICATION

(11) **EP 0 347 365 B1**
(45) Date of publication and mention of the grant of the patent: **28.08.1996**
(21) Application number: 89710057.4
(22) Date of filing: 13.06.1989
(51) Int. Cl.: C12N 7/00, C12N 15/49, A61K 39/395, C12N 5/00

(54) **HIV-2 virus variants**
HIV-2-Virusvarianten
Variants du virus HIV-2

(30) Priority: 14.06.1988 DE 3820223
(43) Date of publication of application: 20.12.1989
(60) Divisional application: 95100149.4
(73) Proprietor: QIAGEN GmbH, 40724 Hilden (DE); CHEMOTHERAPEUTISCHES FORSCHUNGSINSTITUT GEORG-SPEYER-HAUS, D-60596 Frankfurt (DE)
(72) Inventor: Henco, Karsten, Dr., D-4006 Erkrath 2 (DE); von Briesen, Hagen, D-6242 Kronberg (DE); Immelmann, Andreas, Dr., D-4000 Düsseldorf 12 (DE); Kühnel, Herbert, Dr., D-6073 Egelsbach (DE); Dietrich, Ursula, Dr., D-6236 Eschborn (DE); Rübsamen-Waigmann, Helga, Prof. Dr., D-6232 Bad Soden/Taunus (DE); Adamski, Michalina, D-6000 Frankfurt 71 (DE)
(74) Representative: Werner, Hans-Karsten, Dr.Dipl.-Chem.

(56) References cited:
- EP-A- 0 269 520
- EP-A- 0 327 801
- WO-A-89/09815
- NATURE, vol. 328, 6th August 1987, pages 548-550; S.K. ARYA et al.: "New human and simian HIV-related retroviruses process functional transactivator (tat) gene"
- PROC. NATL. ACAD. SCI. USA, vol. 86, April 1989, pages 2383-2387; H. KÜHNEL et al.: "Molecular cloning of two West African human immunodeficiency virus type 2 isolates that replicate well in macrophages: A Gambian isolate, from a patient with neurologic acquired immunodeficiency syndrome, and a highly divergent Ghanian isolate"
- AIDS FORSCHUNG 2, 572-575;
- AIDS RES. HUM RETROVIRUSES 3, 3-10
- J LEUKOCYTE BIOL 37, 407-422
- MICROBIOL REVIEWS 57, 183-289
- PATHOBIOLOGY 60, 213-218

## Description

The present invention relates to HIV-2 virus variants, namely Virus HIV D194 that may be cloned from the corresponding virus isolate HIV D194 (ECACC V 87122303) or from the infected cell line HUT 194 (ECACC V 87122306).

"Molecular cloning of two West African human immunodeficiency virus type 2 isolates which replicate well on macrophages: a Gambian isolate from a case of neurologic aquired immunodeficiency syndrome, and a highly divergent Ghanesian isolate" (Kühnel, H., v. Briesen, H., Dietrich, U., Adamski, M., Mix, D., Biesert, L. Kreutz, R., Immelmann, A., Henco, K., Meichsner, Ch., Andreesen, R., Gelderblom, H. & Rübsamen-Waigmann, H., 1989, Proc. Natl. Acad. Sci. 86, 4, 2383-2387.

In diagnostics, two criteria are demanded to be met, namely specifity and sensitivity for the antigen to be detected. In the diagnostics of AIDS the demand for specifity can certainly be complied with by using the isolates HTLV-III_{B} and LAV-2 (Guyader, M. et al., "Nature" 326, 1987, 662-669) in order to delimit HIV infections from other infections and, thus, to make a rough assignment into the classes of "HIV-2-related infections" or "HIV-1-related infections". However, a problem is constituted by the sensitivity of the diagnosis. In the range of the so-called seroconversion, i.e. the initial occurrence of the antibody in the infected person, a reduction in sensitivity implies an increase in the number of "falsely negative" test results. Accordingly, it is one main goal to shorten the period between an infection and the detectability of this infection as much as possible by improving the test sensitivity.

A decreased cross reactivity, in the practice of the widely employed ELISA diagnostics, is manifested, for example, in a reduced sensitivity. Thus, the use of the described HIV-1 isolate means about an average reduction of the test sensitivity against HIV-2 sera by the factor of 100 to 1000, whereas the isolate HTLV-III_{B} enables almost no detection to be accomplished anymore.

A disastrous principle of the diseases caused by HIV resides in the fact that there is not only one type of each of HIV-1 and HIV-2 virus phenotypes and genotypes. What is to be premised is rather a large group of related viruses, possible even populations which by no way are strictly separated from each other but continuously penetrate one another and undergo some evolutionary development to a more and more increasing divergence, while at the same time they begin by recombination events to exchange between each other parts of the genom. Thus, the existing HIV species form a broad continuous population level in which there are no narrowly delimited subpopulations of one virus variant. There is rather to presumed that a continuum exists which is subject to permanent fluctuations with time.

The classified virus variants HIV-1 and HIV-2 are representatives of the diffusely delimited subpopulations having a relative low degree of relationship, which is manifested by only a partial cross reactivity. On the other hand, there are variants of the HIV-1 group (Rübsamen-Waigmann, H. et al., "AIDS-Forschung" 10, 1987, 572-575; Rübsamen-Waigmann, H. et al., J. Med. Virol. 19, 1986, 335-344; v. Briesen, H. et al., J. Med. Virol. 23, 1987, 51-66), which do significantly stronger cross-react with HIV-2 than the first characterized HIV-1 isolate itself (Hahn, B. et al., "Nature" 312, 1984, 166-169). A commercial product consisting of such an isolate diagnoses distinctly more sera as being HIV-2 positive than does the described standard isolate HTLV-III_{B}.

An ideal diagnostic or therapeutic product should contain at least one representative from the populations as significantly biologically distinguished from one another.

HIV-1 viruses in a multitude of highly polymorphic genetic mutants may cause different diseases such as ARC, LAS, AIDS and encephalopathies (ARC: AIDS-related complex, LAS: lymphadenopathy syndrome, AIDS: acquired immune deficiency syndrom). Cloned virus variants are distinguished in sequence and restriction pattern, even if they have been isolated at the same time, at the same place and even from the same patient (Rübsamen, H. et al., 1986). It could be shown that virus variants of the HIV-1 type are distinguished in some virus antigens up to about 15%. HIV-2's are even different in more than 40% of the aminoacids in some antigens, substitutions, insertions and deletions having been considered (Guyader, M. et al., 1987; Rabson, A.B. & Martin, M.A. "Cell" 40, 1985, 477-480).

EP 0 269 520 deals with retroviruses identified as HIV-2 further referred to as HIV-2_{ROD}.

The present invention provides a variant of the HIV-2 virus. The variant was isolated from a patient suffering from terminal so-called neuro-AIDS. The virus isolate proved to be diagnostic agents, relative to DNA/RNA as well as relative to the virus antigens, for serologically and directly identifying infections by the type HIV-2 in the pre-AIDS and AIDS stages. The variant prefers cells which are derived from myeloidic lines for in vivo replication.

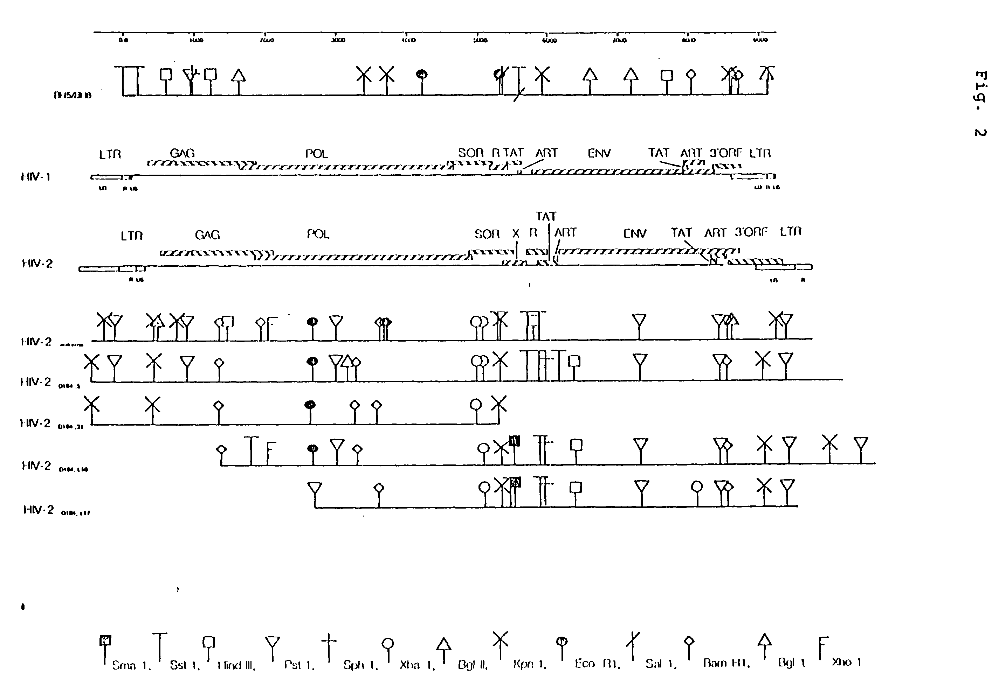

The virus isolate according to the invention comprises viruses and proviruses, the characteristics of which are identical to those of the disclosed restriction map and the sequence of the cloned partial regions (Figures 2-5) . Moreover, the virus isolate comprises a variant which is distinguished from the viruses and proviruses described above in that they are different in their nucleotide sequences from the above-described viruses only by up to 5%, and preferably by 2%, particularly preferred by 1%.

The virus variant according to the invention may cause serious neurological disorders (encephalopathies).

Cloned DNA sequences can be derived from the isolates which are capable of hybridizing with genomic RNA and DNA of the virus variant. Stable gene probes containing such DNA sequences can also be derived which are suitable for the detection of hybridization of those and other HIV variants or related viruses or DNA proviruses in samples to be investigated, more particularly biological or semi-synthetic samples.

It is also possible to derive a preferred embodiment of the invention a virus variant the RNA/DNA of which or respective fragments will hybridize to the virus variant according to the invention under stringent conditions, more particularly c-DNA, genomic DNA, recombinat DNA, synthetic DNA or fragments thereof. These are understood to include variants or fragments which exhibit deletions and insertions in comparison to the virus variants according to the invention.

Stringent conditions of hybridization and washing are meant to be understood as those conditions which ensue by way of experiment or calculation if the melting point of the 100% homologous nucleic acid complexes in conditions of hybridization and washing will be fallen below by not more than 5°C under the buffer conditions employed.

Also claimed according to the invention are cloned synthetic gene probes which may be derived from the above-described virus variants and can be augmented in vector systems in eukaryotes or prokaryotes. In particular, therefore full length cDNA of the virus isolate can be used differing in its nucleotide sequence only by up to 5%. The described cloned DNA fragments are suitable for hybridization with complementary nucleic acids (DNA/RNA) for the purpose of diagnostic detection of the virus variant. The diagnostic tests according to the invention are carried out by using DNA or RNA probes. The probes are radioactive or have been labelled with fluorescent bio- or chemiluminescent groups or enzymes or are specifically detectable with enzymes via coupled reaction systems. The hybridizations may be effected in a homogeneous phase of a solution or in a heterogeneous phase with solid-immobilized nucleic acids, while the solid may be a membrane, particle, cell or tissue, so that the hybridization may also be effected in situ.

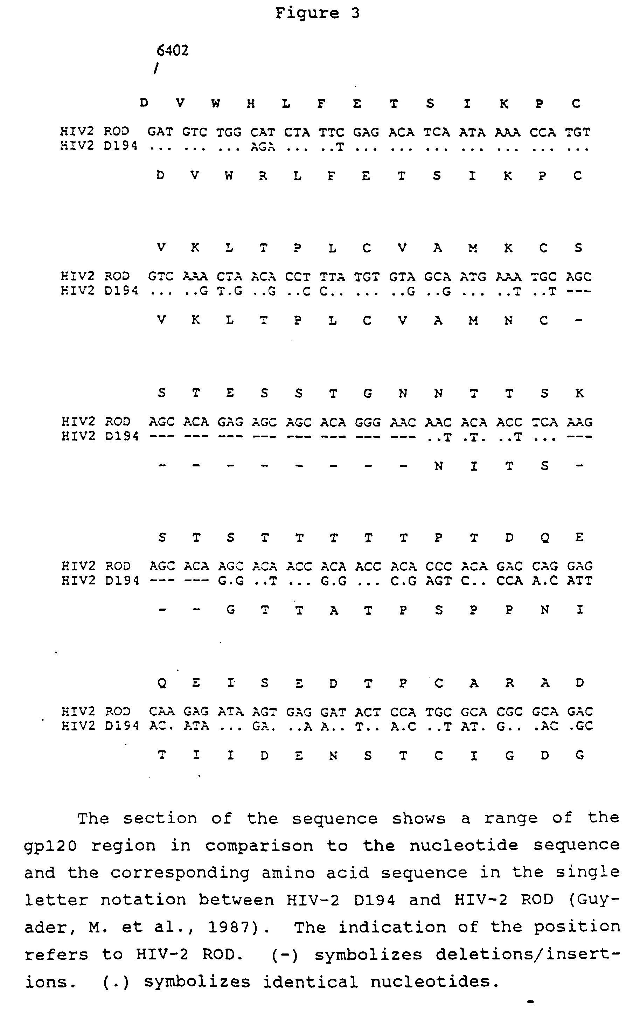

From the virus isolate claimed according to the invention, the corresponding DNA sequences (Figure 2) may be cloned in E. coli bacteria by establishing a genomic lambda-gene bank, starting from the DNA of the lymphocytes infected with the virus isolate. The desired clones are obtained by carrying out a plaque-screening with STLV-III sequences of the gag-pol range. In a more specifical way, there may be used as a probe a DNA derived from the published sequence HIV-2 ROD (Guyader, M. et al., "Nature" 326, 1937, 662-669), or a DNA probe derived from the partial sequences of the isolate HIV-2 D194 according to the invention. Thus from Figure 3 a probe may be derived which under stringent conditions will hybridize only with variants of the type HIV-2 D194, however not with variants of the type HIV-2 ROD.

The diagnostic method based on the use of the viruses claimed according to the invention comprises the following steps: Extraction of RNA or DNA from biological samples, possibly enzymatic processing by restriction enzymes, separation by gel electrophoresis and/or direct blot methods for nucleic acid-binding carriers, and subsequent hybridization with parts of the cloned fragments of the claimed viruses. Hybridizations may also be directly carried out in chemically treated cells or tissues. Therein the origin of the tissues or liquids is insiginificant.

Specifically, a process for the in vitro detection of antibodies against expression products of the viruses of the present invention is characterized in that the expression products or parts thereof of the viruses are detected by means of immunological methods. The process is characterized in that the expression products are proteins, peptides or parts thereof which have been coded within the meaning of an open reading frame on the DNA of the proviral partial sequences as characterized in claim 1 and are prepared by synthetic or biosynthetic processes.

The process is further characterized in that previously a definite amount or a combination of expression products or parts thereof are fixed on microtiter plates, whereupon subsequently biological samples, diluted or undiluted, are contacted with the coated microtiter plates and after incubation and sequential washing steps can be identified by means of a detecting reagent or of labelled anti-HIV antibodies.

Alternatively, filter strips and plastic strips or rods are used instead of microtiter plates, wherein the expression products of the viruses have been fixed at respective specific positions by isolated application of the different antigens.

The expression products or parts thereof can also be separated by gel electrophoresis and then transferred by blotting whereupon incubation with anti-HIV antibodies and the detection thereof are effected. Detection is effected on solid phase carriers to which the antigen determinants have been bonded, with the solid phase carrier consisting of particles.

Expression products can be virus antigens derived from in vitro-infected cells, said antigens being contacted with biological test materials as antigens bonded to fixed cells, and that the subsequent antibody bonding can be determined with immunological detection reagents by means of an apparatus, for example with a cytofluorimeter, or visually.

The antigens can be determined by competitive ELISA. HIV-related nucleic acids (DNA and RNA) can be detected in biological samples, cells and in isolated form by using the nucleic acids according to the present invention.

Expression products can be supplemented by materials which are related to other HIV variants, which, however, are distinguished in their biological properties from the materials of the isolates of the present invention.

For diagnostic and therapeutic goals the described DNA segments may also be employed for expressing coded antigens, parts thereof or combinations thereof with alien antigens. Therein the DNA segments under aimed control of regulation sequences are introduced into pro- or eukaryotic target cells, tissues or multiple-cell organisms to stimulate these to produce the accordingly coded antigens, parts thereof or combinations thereof with alien antigens. Antigens can be detected via the reaction with Anti-HIV-2 antibodies, more particularly from the sera of the respective patients. Antigens having longer open reading frames (>50 amino acids) lend themselves as well those which are subject to splicing processes on the RNA level and are only thus composed to form the longer open reading frames.

Polypeptides originating from the cloned virus variants according to the invention can be used to detect such antigens in the material under investigation which contain similar antigen determinants and thereby do immunologically cross-react. This is particularly suitable for the diagnosis of AIDS and pre-AIDS of virus carriers or asymptomatic virus carriers or virus products, respectively, which are derived from blood. Also the serological detection of the antibodies directed against these antigenic polypeptides as expression products of the viruses claimed according to the invention becomes possible by employing conventional systems such as ELISA. The immunogenic polypeptides may be used as protective polypeptides as vaccines to cause protection against AIDS infections.

The polypeptides according to the invention are understood to include fragments which are intentionally obtained by means of gene-technological methods, starting from longer open reading farmes as well as those obtained by proteolytic enzymes in the production bacterial strains or in vitro by the use of proteases.

The virus isolates according to the invention and the products derived therefom may be combined with other isolates of the partial population HIV-2 in test systems, that is with those which are as far remote as possible in the described population level such as for example, the isolate HIV-2 ROD (Guyader, M. et al., 1987). Thereby it becomes possible sensitively to detect also populations of remote relationship in one test.

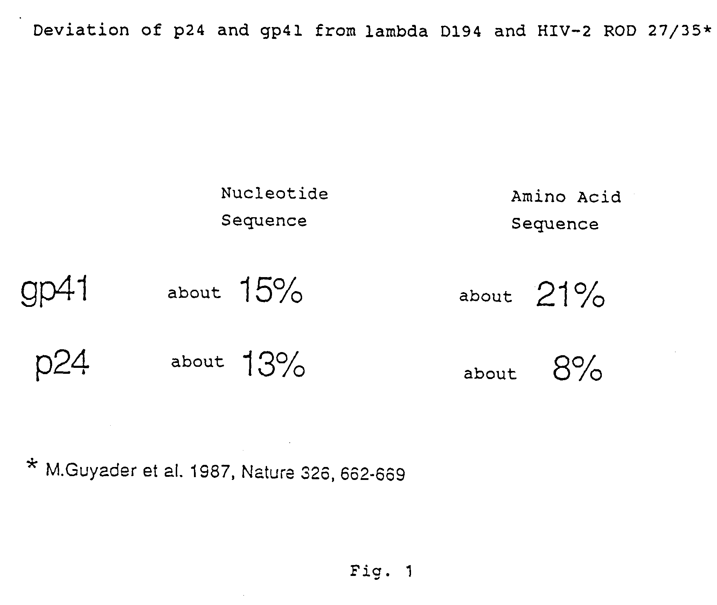

The virus variant according to the invention is highly different from the spectrum of the HIV-1 variants and has a closer molecular relationship to the HIV-2 virus described by Guyader, although it is distinguished therefrom to a significant extent (Figure 1, Figure 2, Figure 3) . Also the biological properties are clearly distinguished from the described HIV-2 isolate. Thus, the variant according to the invention, for the effective in vitro replication, prefers cells which are derived from myeloidic lines. On the contrary, the virus poorly reproduces itself on lymphocytic lines.

The virus HIV D194 according to the invention exclusively caused encephalopathic symptoms in the infected patient, due to which the patient also deceased after an extremely short time and after a fulminant progress of the disease. Samples of the virus claimed according to the invention has been deposited in the form of its isolate at the European Collection of Animal Cell Cultures under the designations HIV D194 (Accession No. V 87122303).

A cell line infected with the virus isolate HIV D194 has been deposited under the designation HUT 194 (ECACC V 87122306) at the above-identified Deposit.

Figure 1 shows the deviation of the proteins p24 and gp41 from lambda D194 and HIV-2 ROD 27/35 in its nucleotide sequence and amino acid sequence (Guyader, M. et al., 1987, Nature 326, pages 662 - 669).

Figure 2 shows the restriction maps of the virus isolate according to the invention in comparison to known HIV sequences.

Figure 3 shows a comparative section of a sequence between HIV-2 ROD (Guyader, M. et al., 1987) and HIV-2 D194, which demonstrates the significant divergence of the variant HIV-2 D194 according to the invention in a coding range of the envelope protein gp120.

The section of the sequence shows a range of the gp120 region in comparison to the nucleotide sequence and the corresponding amino acid sequence in the single letter notation between HIV-2 D194 and HIV-2 ROD (Guyader, M. et al., 1987). The indication of the position refers to HIV-2 ROD. (-) symbolizes deletions/insertions. (.) symbolizes identical nucleotides.

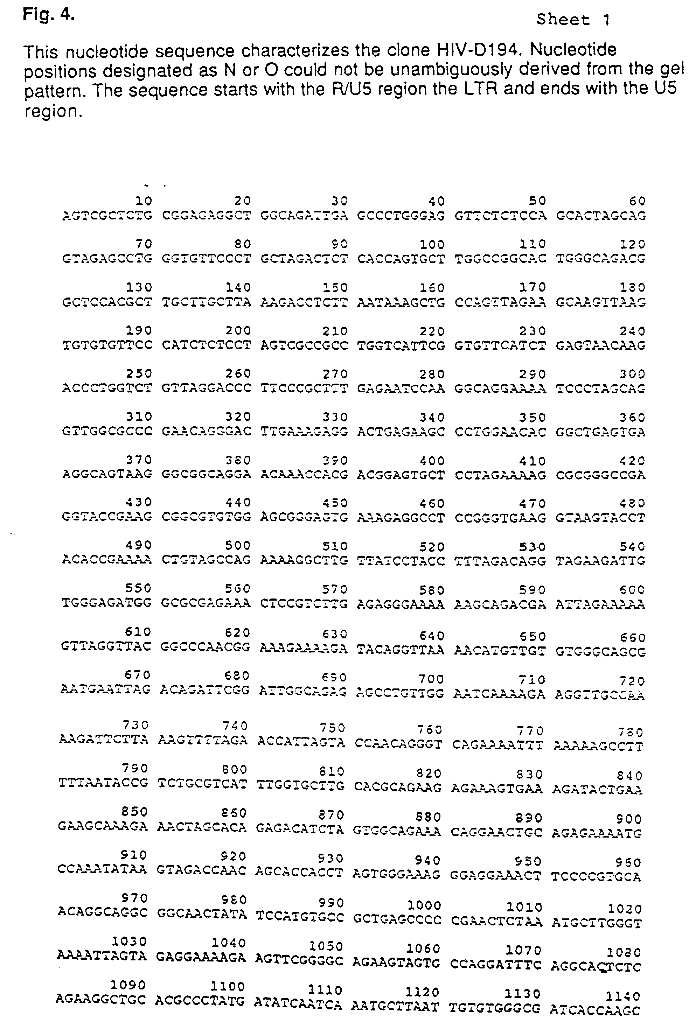

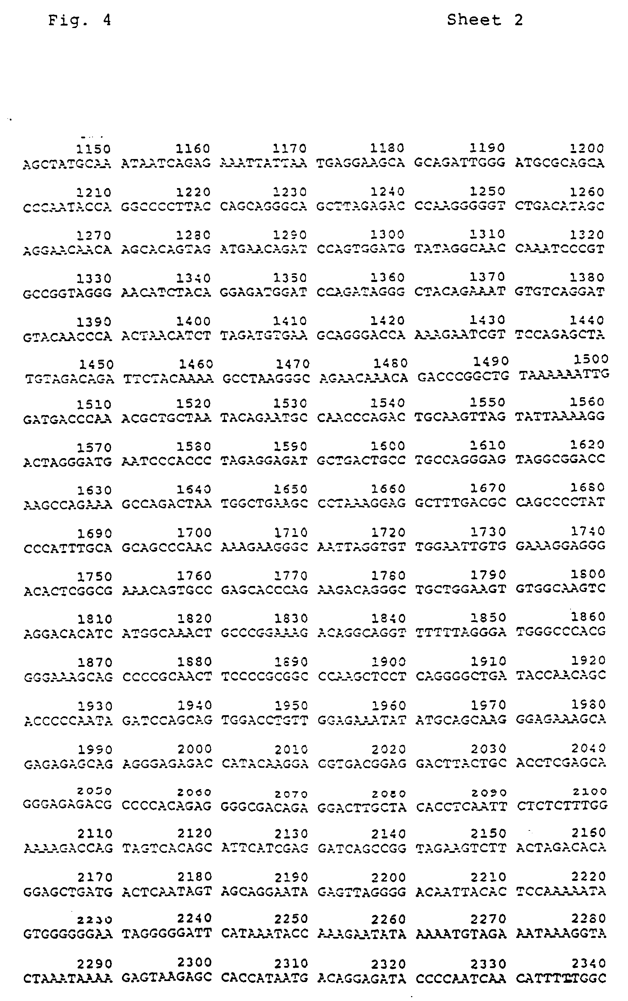

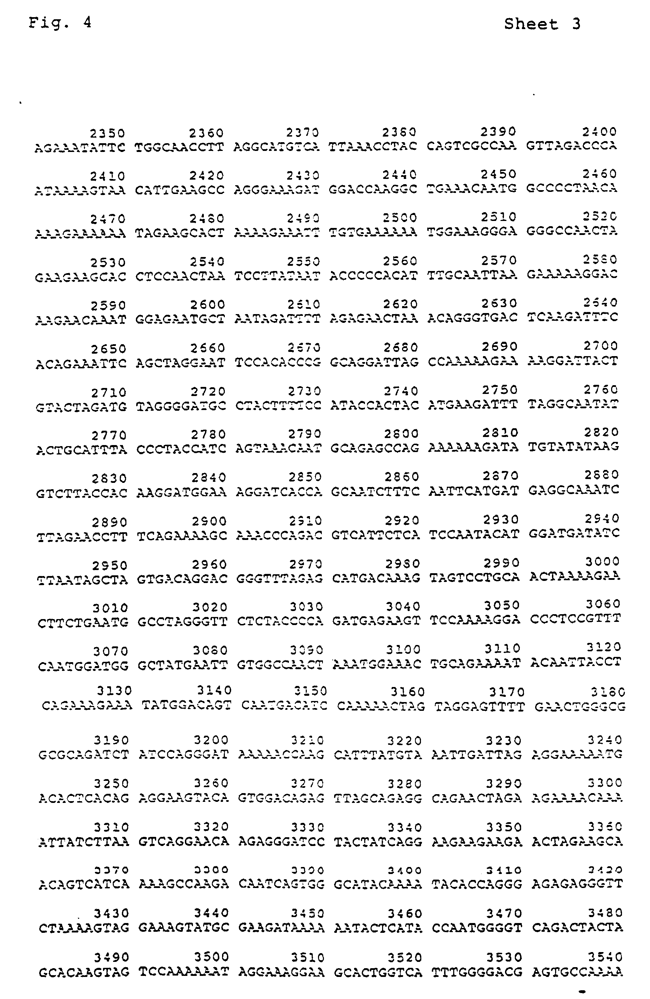

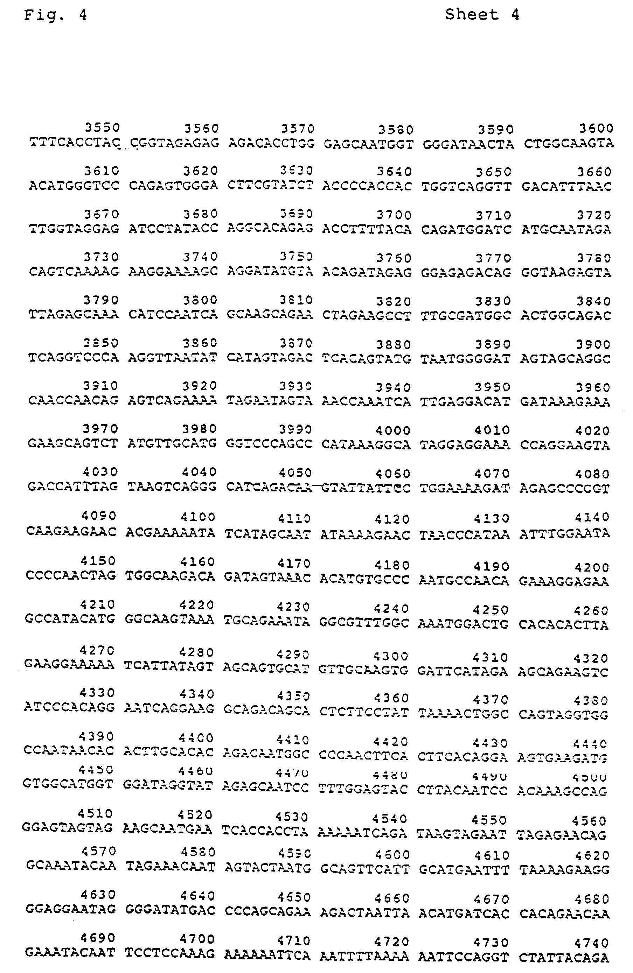

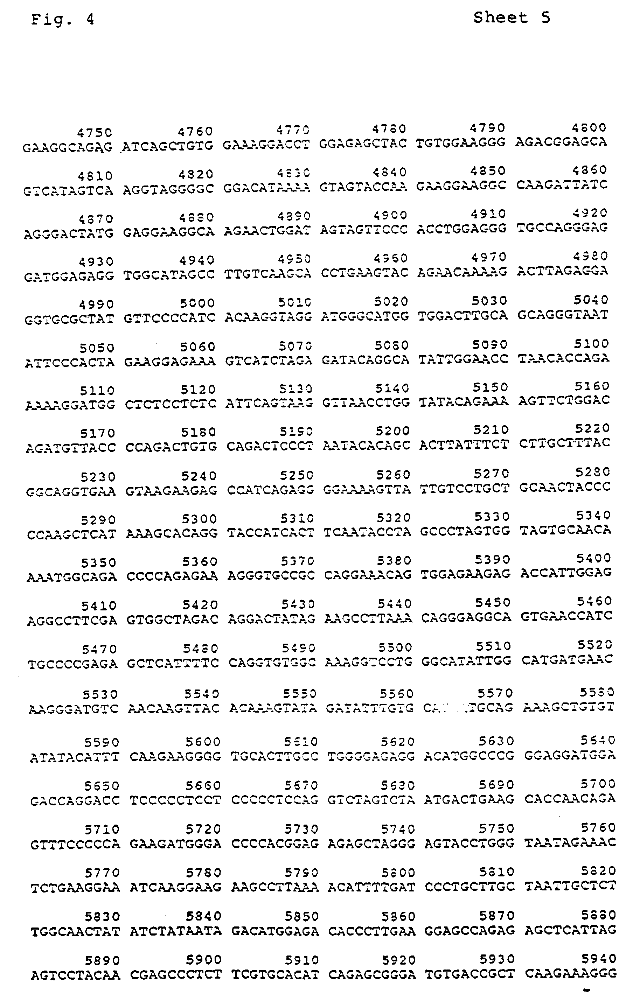

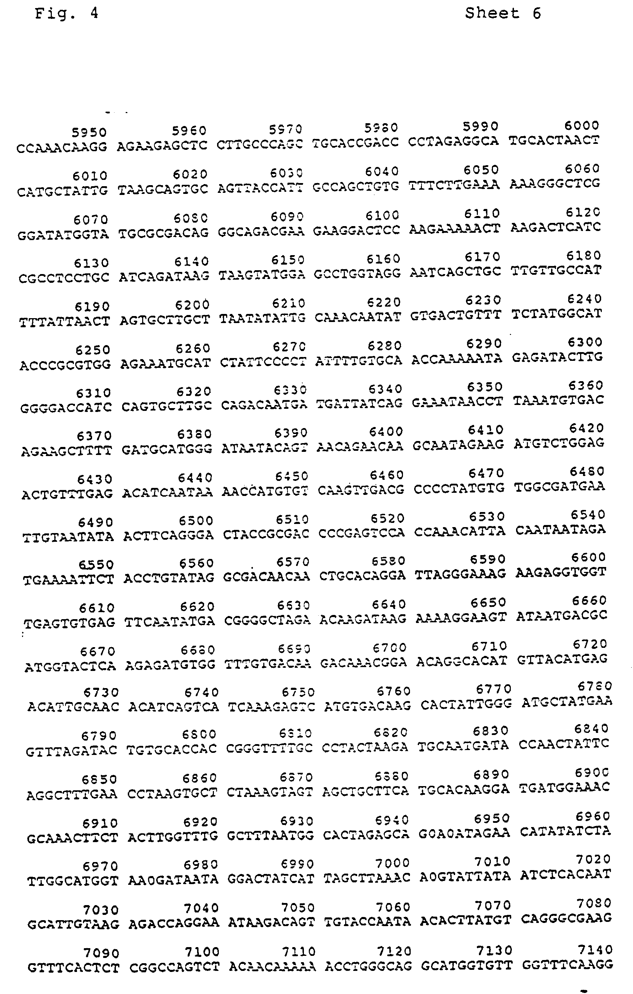

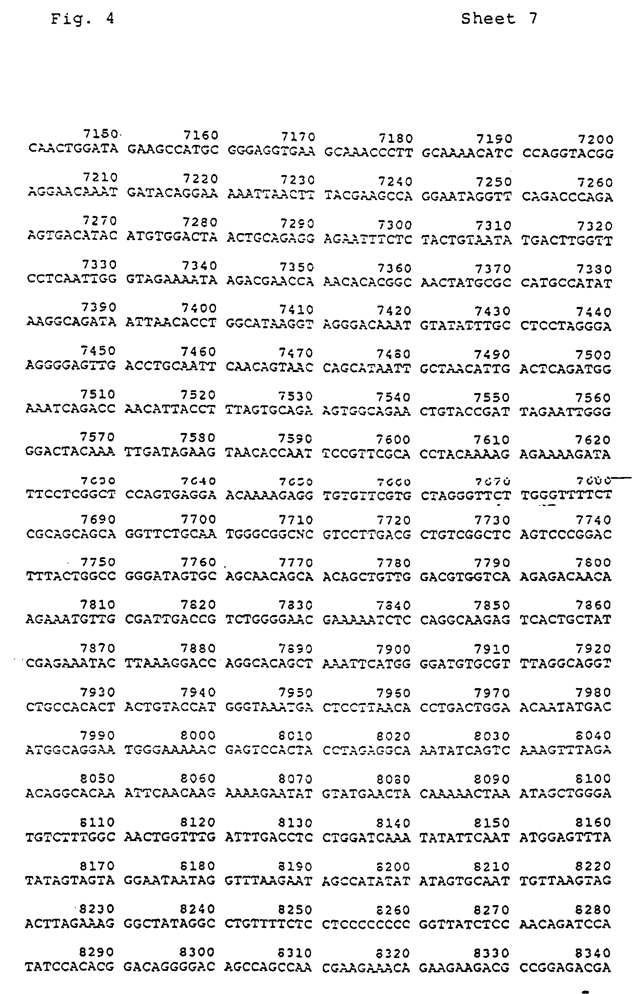

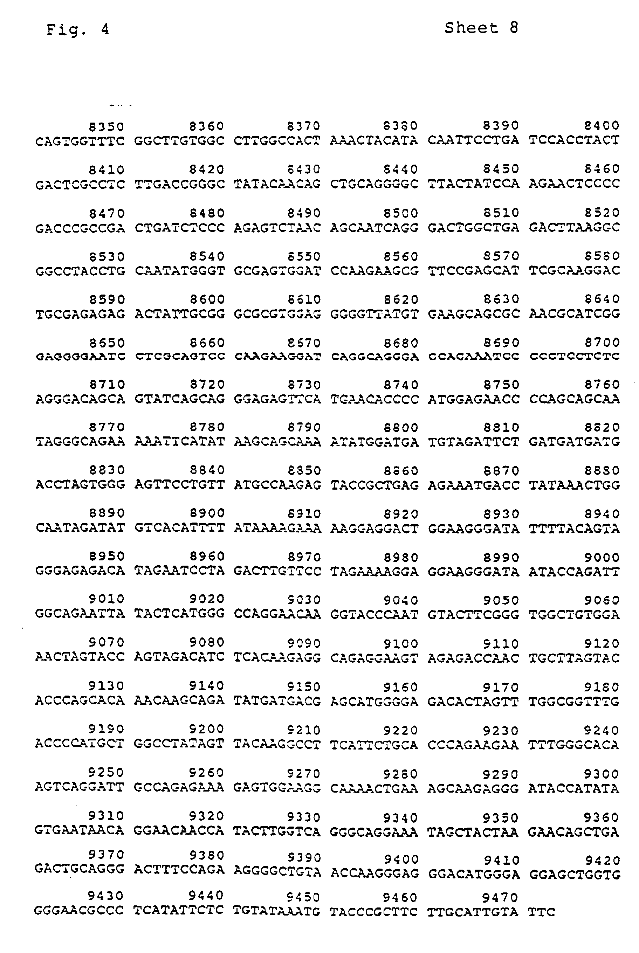

Figure 4 shows a nucleotide sequence, characterizing the clone HIV-D194. Nucleotide positions designated as N or O could not be unambiguously derived from the gel pattern. The sequence starts with R/U5 region the LTR and ends with U5 region. The sequence shown is derived from subclone L10 (see restriction map). This clone differs from others derived from the same patient/blood sample by around 1% in the nucleotide sequence as it was determined by comparison with 5kb homologous sequences derived from clone HIV-194,5.

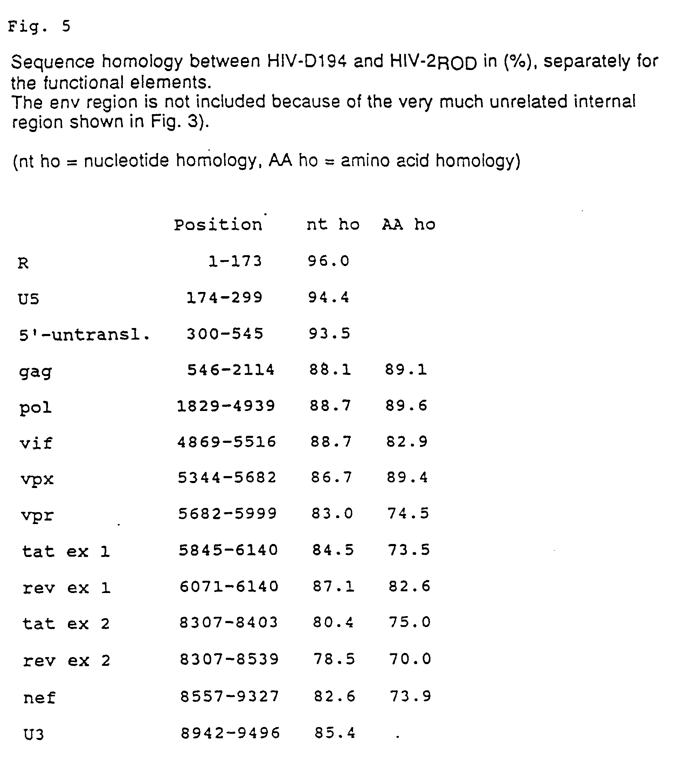

Figure 5 shows the sequence homology between HIV-D194 and HIV-2 ROD in (%), separately for the functional elements.

Experimental results and characteristics of HIV-D194 are described in Kühnel, H. et al. (1989) Proc. Natl. Acad. Sci. 86, 4, 2383-2387.

The sequence of HIV-D194 shows a lot of so-called "open reading frames". Most of these reading frames can be related to in vivo expressed proteins/antigens by comparison of homologies to previously described HIV-viruses, by comparison of Western blots performed with HIV-D194 antigens derived from infected HUT78 or U937 cells and by probing with sera from the corresponding patients and reference sera.

Other open reading frames are not identified on the level of their expressed antigens defined by function or antibody staining on Western Blot. However, they can be expressed under some circumstances in vivo. Other reading frames, even short ones, can be expressed as well in a way difficult to predict solely on the basis of nucleic acid sequencing data because of splicing processes.

Antigenic determinants on expressed proteins as they are important for the biological function, for target antigens in diagnostics or for immunization are spread all over the expressed linear protein sequence. Parts of these sequences can have more general antigenic properties than others as can be shown by peptide screening/mapping for antigenic sites. These sites can be expressed as single epitopes or as continuous polypeptide or in a version of in vitro or synthetically spliced antigens. Antigenicity of the expressed products can be demonstrated by antigen fixation and blotting in the Western Blot assay. Constructions for antigen expression in E. coli can be done by using conventional techniques using synthetic genes, restriction fragments from cloned viral genome segments, trimming products thereof by using exonuclease or DNase I or by using sequence specific synthetic primers defining the desired 5' and 3' end of the fragment to be expressed together with appropriate restriction sites. These restriction sites can easily be used for ligation into a panel of expression vectors of different organisms like those derived from PLc24 (Remault et al. 1981 Gene 15, 81-83) with multicloning sites (pEX).

The expressed antigens were shown to specifically react with patients' sera. The antigenic sequence corresponding to the region shown in Figure 3 is highly specific for this particular subfamily of HIV-variants.

### EXAMPLE

Cloned subfragments such as the Kpn-Kpn fragment comprising the gag-pol region of HIV-D194 are used as probes for HIV-2 type and SIV type sequences by hybridizing under conditions 30-40°C less in hybridization and washing conditions appropriate for homologous sequences.

HIV-1 sequences do not show up in blot and in situ hybridization unambiguously, although this region contains the p24/27 coding region which heavily cross-reacts with anti HIV-1 sera. A nucleic acid probe such as shown in and corresponding to Figure 3, however, highly specifically detects the specific subfamily of HIV-D194 compared to all other known HIV isolates. This is shown by in situ hybridization using run-off RNA of this particular region.

## Claims

1. A virus isolate HIV-2 D194 (ECACC V 87122303) which prefers cells which are derived from myeloidic lines for in vitro replication.

2. DNA of the proviral partial sequences according to claim 1 having the following restriction endonuclease section-site characteristics

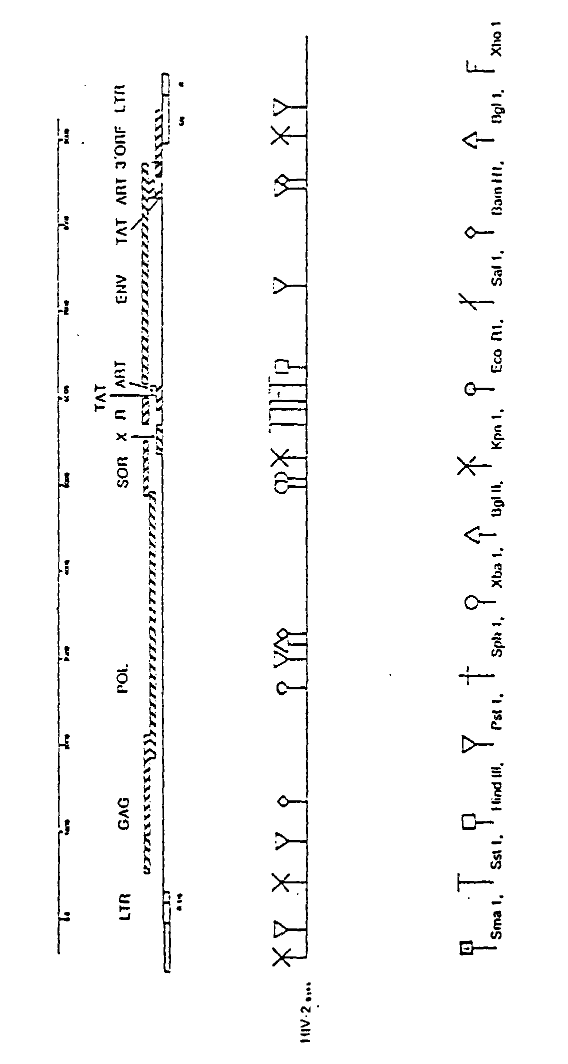

3. cDNA of the virus isolates according to claim 1 differing in their nucleotide sequences only by up to 5%.

4. Recombinant DNA containing DNA according to claims 2 or 3.

5. Cells which have been transformed with nucleic acids according to any one of claims 2 to 4.

6. Cells which have been infected with virus isolates according to claim 1.

7. Cell line HUT 194 (ECACC V 87122306)

## Patentansprüche

1. Virusisolat HIV-2 D194 (ECACC V 87122303), das zur in-vivo-Replikation Zellen bevorzugt, die von myeloiden Linien abstammen.

2. DNA der proviralen partiellen Sequenzen gemäß Anspruch 1, die die folgenden Merkmale in bezug auf Restriktions-Endonuclease-Schnittstellen aufweist:

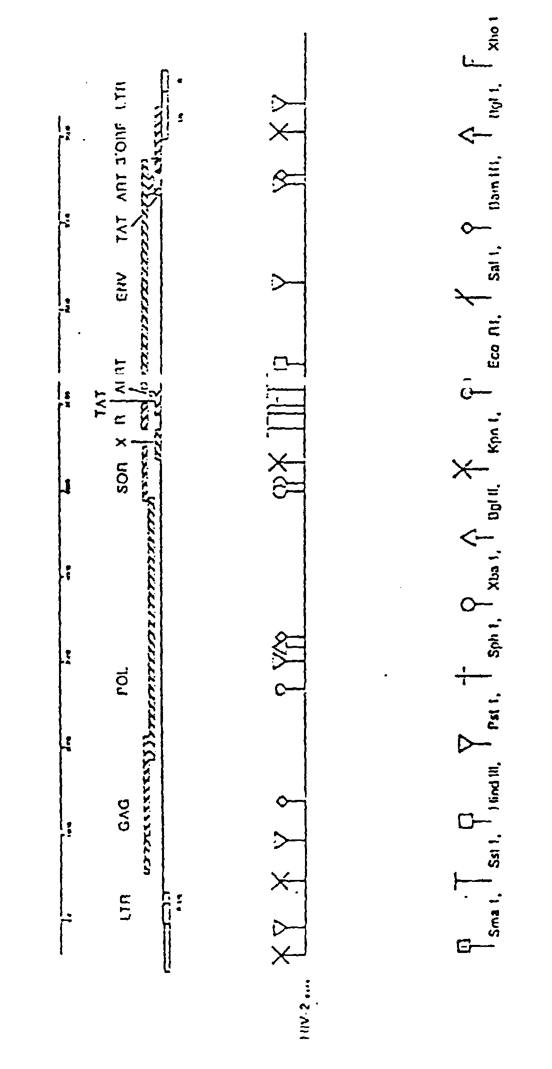

3. cDNA der Virusisolate gemäß Anspruch 1, die sich in ihren Nucleotidsequenzen nur um bis zu 5% unterscheidet.

4. Rekombinante DNA, die DNA gemäß den Ansprüchen 2 oder 3 enthält.

5. Zellen, die mit Nucleinsäuren gemäß einem der Ansprüche 2 bis 4 transformiert wurden.

6. Zellen, die mit Virusisolaten gemäß Anspruch 1 infiziert wurden.

7. Zellinie HUT 194 (ECACC V 87122306).

## Revendications

1. Un isolat de virus HIV-2 D194 (ECACC V 87122303) qui préfère les cellules dérivées de lignées myéloïdiques pour la réplication in vitro.

2. ADN des séquences partielles provirales selon la revendication 1, ayant les caractéristiques suivantes de site de section par l'endonucléase de restriction

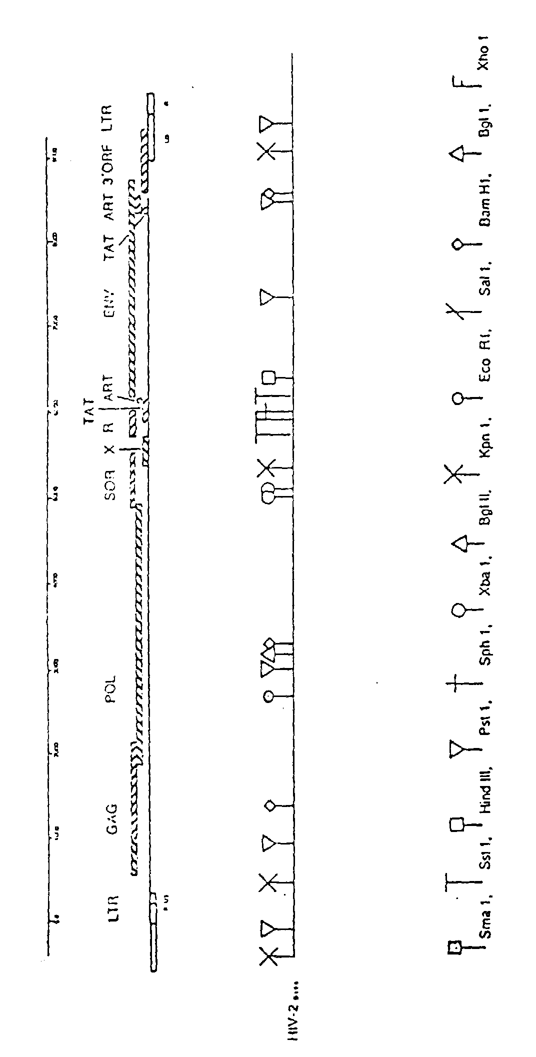

3. ADN complémentaire des isolats de virus selon la revendication 1, dont les séquences de nucléotides diffèrent de seulement 5 % au maximum.

4. ADN recombinant contenant de l'ADN selon la revendication 2 ou 3.

5. Cellules qui ont été transformées par des acides nucléiques selon l'une quelconque des revendications 2 à 4.

6. Cellules qui ont été affectées par des isolats de virus selon la revendication 1.

7. Lignée cellulaire HUT 194 (ECACC V 87122306)
